(19) **Europäisches Patentamt**

**European Patent Office**

**Office européen des brevets**

(11) **EP 1 731 528 A1**

(12) **EUROPEAN PATENT APPLICATION**
published in accordance with Art. 158(3) EPC

(43) Date of publication:
**13.12.2006 Bulletin 2006/50**

(51) Int Cl.:
***C07K 1/14*** (2006.01)

(21) Application number: **05721599.8**

(22) Date of filing: **28.03.2005**

(86) International application number:
**PCT/JP2005/005672**

(87) International publication number:
**WO 2005/095439 (13.10.2005 Gazette 2005/41)**

(84) Designated Contracting States:
**DE FR**

(30) Priority: **31.03.2004 JP 2004107286**

(71) Applicant: **Tokyo University of Agriculture and Technology Tlo Co., Ltd.
Tokyo 184-8588 (JP)**

(72) Inventors:
• **NOMURA, Y.
AGRICULTURE DEP.TOKYO UNI. OF AGRI&TECH
Tokyo 1838509 (JP)**
• **AIHARA, Michiro
2020013 (JP)**

• **NAKAJIMA, Daiki
1830053 (JP)**
• **KENJOU, Seiji;
C/O TOYO FEATHER INDUSTRY CO.,LTD.
2290006 (JP)**
• **TSUKUDA, Mitsuaki;
TOYO FEATHER INDUSTRY CO., LTD.
2290006 (JP)**
• **TSUDA, Yuuiti;
C/O TOYO FEATHER INDUSTRY CO., LTD.
2290006 (JP)**

(74) Representative: **Hartz, Nikolai
Wächtershäuser & Hartz
Patentanwälte
Weinstrasse 8
80333 München (DE)**

(54) **PROCESS FOR PRODUCING SOLUBILIZED KERATIN**

(57)    A process for producing a solubilized keratin of utility value easily with high yield while suppressing coloration and smelling occurring in the hydrolysis of keratin. There is provided a process for producing a solubilized keratin, **characterized in that** it comprises regulating the water content of keratin raw material to a hydrous state of 20 to 80%, hydrolyzing the same in an alkali solution, neutralizing the hydrolyzate liquid, and extracting a keratin hydrolyzate from the supernatant.

EP 1 731 528 A1

**Description**

Technical Field

The invention relates to a process for producing solubilized keratin by use of a material, such as animal hair, containing keratin.

Background Art

Keratin is a protein giving rise to the formation of the hair, nail, horn and skin existing on the body surface of an animal. In recent years, keratin and its hydrolyzate have been reported to be applicable to animal feeds, fertilizers, base materials for cosmetics, and other surfactants and medical high-molecular materials suited to ecological system. Such reports led to many researches intended to extract keratin from wool and feathers.

Keratin is extremely difficult to extract, because it is a hardly soluble high-molecular substance having a natural crosslinking structure. The method for solubilizing keratin consists roughly of two steps. The first step is characterized by pulverizing keratin materials. In order to substantiate it, there are many methods, such as a method of suspending the keratin material in various solvents and then crushing in a dry or wet pulverizer (see patent documents 1 to 3) ; a method of crushing in liquid nitrogen (see patent documents 4 and 5); a method of crushing at high pressure; and a method of cutting by cutter and then crushing in a dry or wet pulverizer (see patent document 6). The second step is characterized by hydrolyzing pulverized keratin and then solubilizing the resultant. In order to substantiate it, there are some methods including a method of using protein denaturing agent or enzyme in reducing condition (see patent documents 7 to 14), and a method of using acid or alkali (see patent document 15).

However, the conventional solubilizing method consisting of these two steps is not free from the disadvantage that the solubilized keratin is hardly possible to use at high concentration because of its peculiar smell or color. This is a serious obstacle to the use solubilized keratin as a hair-protective agent or the like. Wool or feathers is well known to have a low specific gravity and tends to be entangled itself, so there has been a problem in that its volume becomes bulky, thereby worsening its hydrolysis efficiency.

[Patent document 1] Japanese Patent Application Laid-Open No. 4-281856
[Patent document 2] Japanese Patent Application Laid-Open No. 4-312534
[Patent document 3] Japanese Patent Application Laid-Open No. 2001-302800
[Patent document 4] Japanese Patent Publication No. 61-2416
[Patent document 5] Japanese Patent Publication No. 57-163392
[Patent document 6] Japanese Patent Application Laid-Open No. 5-170926
[Patent document 7] Japanese Patent Application Laid-Open No. 6-336499
[Patent document 8] Japanese Patent Application Laid-Open No. 6-100600
[Patent document 9] Japanese Patent Application Laid-Open No. 6-116300
[Patent document 10] Japanese Patent Application Laid-Open No. 10-291998
[Patent document 11] Japanese Patent Application Laid-Open No. 10-291999
[Patent document 12] Japanese Patent Application Laid-Open No. 7-21061
[Patent document 13] Japanese Patent No. 2777196
[Patent document 14] Japanese Patent No. 3283302
[Patent document 15] Japanese Patent Application Laid-Open No. 2003-301377

Disclosure of the Invention

Problem to be Solved by the Invention

An object of the present invention is to provide a process for producing a solubilized keratin of utility value easily with high yield while suppressing coloration and smelling occurring in the hydrolysis of keratin.

Means for Solving the Problem

The inventors thoroughly investigated manufacturing of decomposed keratin by alkaline hydrolysis, and found that solubilized keratin substantially reduced in deposits of smell, in particular, not causing coloration, can be obtained efficiently, by hydrolyzing keratin raw material having a predetermined water concentration in alkali, neutralizing the treated solution, and extracting keratin from the supernatant.

Thus, the invention relates to a process for producing a solubilized keratin, characterized by comprising regulating the water content of keratin raw material to a hydrous state of 20 to 80%, hydrolyzing the same in an alkali solution, neutralizing the hydrolyzate liquid, and extracting a keratin hydrolyzate from the supernatant.

The invention also relates to a solubilized feather keratin of average molecular weight of 8000 to 13000 (gel filtration method) manufactured by the above method, using feathers as keratin raw material.

Further, the invention relates to a cosmetic ingredient composedof solubilized keratin manufactured by the above method.

Effect of the Invention

According to the method of the invention, the keratin raw material can be hydrolyzed efficiently in moderate conditions, and a hydrolyzate can be obtained without coloration or rarely causing smelling. Thus, the method of the invention is very useful as a method of obtaining a solubilized keratin of high utility value as hair oil or cosmetic ingredient easily and with high field. According to the method of the invention, used materials such as old clothing and feather bedding, which are not reusable for their original purposes, as well as other wool and feather wastes massively incinerated so far can be recycled effectively, thereby contributing greatly to conservation of environment.

Brief Description of the Drawings

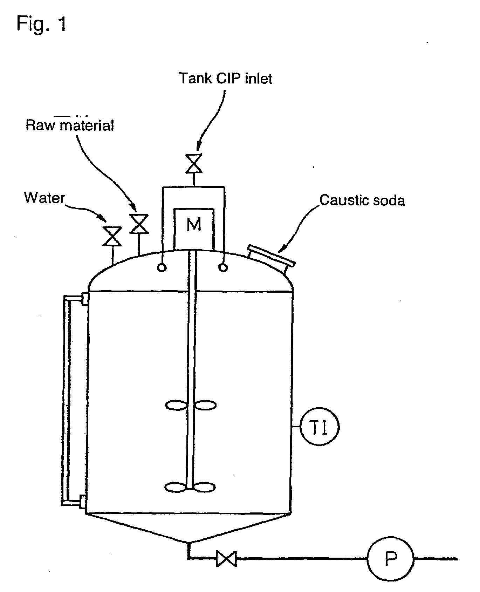

Fig. 1 shows a reaction tank used in keratin solubilizing process.

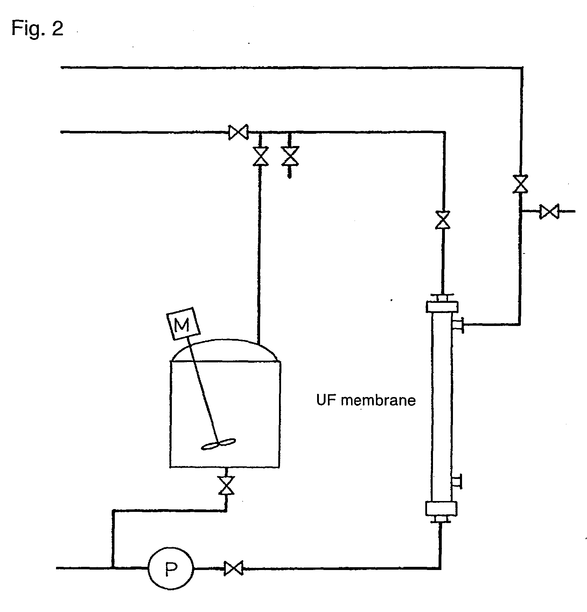

Fig. 2 shows a filter and a concentration tank used in filtering and concentrating solubilized keratin.
Fig. 3 shows results of gel filtration of solubilized keratin.
Fig. 4 shows skin stimulation test results of prepared solubilized keratin, in which a to c show negative control group, positive control group, and test group, respectively, and numerals in the diagram represent concentration of sensitizing substances (1: 2.0%, 2: 1.0%, 3: 0.5%, 4: 0.2%, 5: 0.1%, 6: 0.05%, 7: 0.02%, 8: 0.01%).

Best Mode for Carrying Out the Invention

The process for producing solubilized keratin of the invention is characterized in that it comprises regulating the water content of keratin raw material to a hydrous state of 20 to 80%, hydrolyzing the same in an alkali solution, neutralizing the hydrolyzate liquid, and extracting a keratin hydrolyzate from the supernatant.
In the invention, solubilized keratin is a keratin hydrolyzate obtained by hydrolyzing an arbitrary amide bond in molecules in keratin raw material, and its average molecular weight is 8000 to 13000, preferably 9000 to 12000, and more preferably 10000 to 11000.

Keratin raw material maybe anymaterial containing keratin such as hair, nail, horn and skin of animals. Preferred examples include animal hair from fowl, sheep, alpaca, mohair, angora, cashmere, etc. , and feathers and wool are preferred in particular. Keratin raw materials of the invention include secondary products from these materials, i. e., feather bedclothes such as feather bedding, feather clothing such as down jacket, wool bedclothes such as mouton, and wool clothing such as sweater. From the viewpoint of effective use of resources, it is preferred to use recycled products of them and waste wool and waste feathers discharged from manufacturing factory and poultry farm.

The above-mentioned keratin raw materials can be used directly, but it is preferred to use them after cleaning in water, solvents, or detergents. By cleaning, dirt and pigment on animal hair can be washed away, and smell and coloration of keratin hydrolyzate can be suppressed. It is particularly preferred to use after cleaning when keratin raw materials are wool or feather clothes, bedding, recycled products, waste wool or waste feathers.
Solvents used in cleaning are organic solvents generally used in dry cleaning, including petroleum solvent, chlorine solvent, and fluorine solvent. Detergents are not particularly limited, and include clothes detergents blending anionic surface active agent, amphoteric surface active agent, and nonionic surface active agent. After cleaning by solvent or detergent, it is necessary to rinse sufficiently in order to remove the detergent. After cleaning or aside from cleaning, it is preferred to immerse keratin raw material in alkali solution (sodium hydroxide solution of 0.1 to 0.8 mol/L, etc.), and the hydrophobic surface of keratin raw material is denatured, and it is preferred because affinity for solution is increased in the process of hydrolysis.

Before alkali treatment, grinding or pulverizing of keratin raw material is not particularly required, but when used afterpulverizing, efficiencyof hydrolysis is further enhanced

In the process for producing solubilized keratin of the invention, prior to alkaline hydrolysis of keratin raw material, it is necessary to regulate the water content of keratin raw material to a hydrous state of 20 to 80%. Generally, in feathers or wool, the water content is about 12% at temperature of 20°C and humidity of 65%, but a higher water content is required in the invention. As a result, bulk volume of keratin raw material is decreased, theaffinityof solvent andrawmaterial is improved, and the weight thereof becomes constant, thereby making it possible to perform hydrolysis efficiently in more moderate condition, and at the same time coloration and smell of keratin hydrolyzate can be suppressed. Considering from such effect, the water content of keratin raw material is preferably 20 to 80%, more preferably 25 to 80%, and further preferably 30 to 75%.

Water content can be adjusted by immersing keratin raw material in water, and dehydrating it for a specified time at specific temperature and specific humidity. When the keratin raw material is cleaned, the water content can be adjusted in rinsing and dehydrating processes.

Water content (moisture rate) can be measured from the dry weight loss when a specific amount of raw material is dried for 16 hours or longer in a thermostatic oven at 80°C.

Hydrolyzing process of the invention is carried out in alkali solution, and the usable alkali is any material used in hydrolysis of protein or peptide, including alkali metal hydroxides such as sodium hydroxide and potassium hydroxide, alkali earth metal hydroxides such as calcium hydroxide, and ammonia, which may be selected properly depending on the properties of keratin raw material. When using wool or feathers as raw material, it is preferred to use sodium hydroxide and potassium hydroxide in terms of reaction efficiency, cost and safety.

Alkali solution is preferably aqueous solution of alkali, but a mixed solvent of alcohol and water may be also used.

The alkali concentration in solution may be properly selected depending on the keratin raw material, and when using feathers or wool, it is preferred to be 0.1 to 0.8 mol/L, or more preferably 0.2 to 0.5 mol/L.

Hydrolyzing process is executed by shaking or stirring keratin raw material in the alkali solution. Reaction time is usually 0.1 to 72 hours in the temperature range of 20 to 120°C. In this manner, in the method of the invention, hydrolysis can be performed in a short time at high temperature and at low alkali concentration, and hence occurrence of smell or coloration of hydrolyzate can be suppressed.

When using wool or feathers as raw material, the hydrolyzing condition is preferably alkali concentration of 0.1 to 0.5 mol/L, temperature of 80 to 120°C, and time of 0.1 to 16 hours.

After termination of hydrolyzing reaction, the solution is neutralized. It is preferred to neutralize by using acid and/or peroxide, and acid includes hydrochloric acid, sulfuric acid, andaceticacid, and preferably hydrochloric acid or acetic acid is used, and hydrochloric acid is most preferred. Peroxide includes performic acid, perchloric acid, and hydrogen peroxide, and preferably perchloric acid or hydrogen peroxide is used, and hydrogen peroxide is most preferred. Peroxide is particularly preferred because it has oxidizing power to decompose melanin, pigment, and odorous substance, and has decoloring and deodorizing effects.

Such acid or peroxide may be used either alone, or in proper combination of plural types.

By the neutralizing process, keratin hydrolyzate is separated into keratin solution and undissolved matter, and the solubilized keratin of the invention is obtained by extracting from its supernatant (dissolved solution). Extraction is realized by usual solid-liquid separating means, such as filtration, desalination, and centrifugal separation. Active carbon is used for filtration, and desalination can be carried out by ultrafiltration, ion exchange, dialysis membrane, electric dialysis, electrolytic dialysis, and gel filtration.

The thus obtained solubilized keratin may be directlyused in various applications, or, as required, it may be further refined, or condensed by removing moisture, or prepared in powder form.

The obtained keratin hydrolyzate (solubilized keratin) is almost free from coloration, smell, and skin irritation. In particular, as compared with keratin hydrolyzate obtained by a conventional method, smell is substantially decreased (see examples below). According to the method of the invention, the keratin hydrolyzate of high quality can be manufactured easily and with high yield.

Solubilized feather keratin manufactured by using feathers as the keratin rawmaterial by the method of the invention is a novel protein of average molecular weight of about 8000 to 13000 (gel filtration method), and is colorless, odorless, and free from skin irritation, and it has an excellent hair protective action and moisture retaining property.

The thus obtained solubilized keratin can be prepared in various derivatives depending on the purpose, such as ester derivative, quaternary ammonium derivative, acylated derivative, and sililated derivative, by modifying the functional group in the molecule of keratin hydrolyzate, and known methods may be employed therefor.

Having such properties, the solubilized keratin of the invention is expected to be blended in cosmetics, such as hair cosmetics (for example, shampoo, treatment, hair rinse, hair cream, hair conditioner, hair lotion, hair pack, hair growing and developing agent, hair color, permanent wave solutions), toilet water, lotion, face wash, hand cream, shaving foam, after-shaving foam, depilating agent, bath salts, soap, mascara, and other make-up goods.

The blending amount of solubilized keratin in cosmetics is not particularly specified, as long as it is enough to have hair protective action or add moisture and gloss to hair and skin, and it is usually added by 0.1 to 30 mass% in the cosmetic, or preferably 1 to 20 mass%. The cosmetic additive of the invention composed of solubilized keratin is blended in the cosmetics together with various cosmetic ingredients, including various surface active agents such as anionic surface active agent, cationic surface active agent, amphoteric surface active agent, and nonionic surface active agent, animal and vegetable originated protein hydrolyzates and derivatives thereof such as collagen, keratin, soybean, and silk, synthetic polymers such as polyethylene glycol and propylene glycol, humidity retaining agents such as glycerin and butylene glycol, oil solutions such as animal and vegetable oil, ester oil, fatty acid, higher alcohols and lower alcohols, silicone oil, silane compounds such as silane coupling agent, animal and vegetable originated protein and yeast such as collagen, keratin, casein, soybean, and wheat, microorganism originated protein such as fungi, and hydrolyzate of protein, its derivative, various amino acids such as L-arginine, L-cysteine, saccharides such as glucose, glucosamine, glucuronic acid, chitosan, hyaluronic acid, trehalose, and xyloglucan, preservatives, and perfumes.

Examples

The invention is more specifically described below by presenting examples, but it must be noted that the invention is not limited by these examples alone.
In the examples and comparative examples, the blending of ingredients is indicated by content by mass, and solutions are indicated by solid matter concentration in parentheses after the content. The percentage showing the concentration is the concentration by mass%.

Example 1: Manufacture of solubilized keratin (1)

In 10 g of feathers and water blended 1 : 1 by weight (water content 50%), 300 mL of 0.1 to 1.0 normal sodium hydroxide solution is added, and the solution is shaken and extracted for 24 to 72 hours at room temperature. By this operation, about 30 to 70% of keratin is solubilized. The extraction residue is filtered by centrifuge or filter, and the filtrate is neutralized in hydrochloric acid. The neutralized solution is desalinated by ultrafiltration, freeze-dried, and 1.2 g of solubilized keratin (product 1 of the invention) was obtained.

Example 2: Measurement of molecular weight

Molecular weight of the solubilized keratin prepared in example 1 was measured by high performance liquidchromatography. The condition is as follows. The apparatus is GPC-8030 of Toso, the eluate is purified water, the flow velocity is 0.6 mL/min, and the detection is performed by UV 280 nm and RI. Dextran having known molecular weight was used as the standard. As a result, the average molecular weight was estimated to be 9074.

Example 3: Manufacture of solubilized keratin (2)

Washing 10 g of unground feathers in tap water, the feather weight after dewatering was adjusted to 20 g (water content 50%) . In an Erlenmeyer flask of inner volume of 500 mL, 6 g of sodium hydroxide and 300 g of water were added, 20 g of washed feathers was supplied, and the resultant was stirred for 3 days at 27°C to hydrolyze the feathers. After hydrolysis, undecomposed matter was filtered, and 10 g of 30% hydrogen peroxide water was added. After decoloration for 24 hours at room temperature, 12 g of sodium sulfite was added, and excessive hydrogen peroxide was removed. Further, by desalinating and refining by a dialysis membrane, the solution was freeze-dried, and 4.2 g of solubilized keratin powder derived from feathers (product 2 of the invention) was obtained.

Example 4: Manufacture of solubilized keratin (3)

Washing 20 g of ground feathers in tap water, the feather weight after dewatering was adjusted to 40 g (water content 50%) . In an Erlenmeyer flask of inner volume of 500 mL, 2.4 g of sodium hydroxide and 300 g of water were added, 40 g of washed ground feathers was supplied, and hydrolyzed for 5 minutes at 120°C. After hydrolysis, undecomposed matter was filtered, and 21.4 g of 30% hydrogen peroxide water was added. After decoloration for 24 hours in 37°C thermostatic oven, 24 g of sodium sulfite was added, and excessive hydrogen peroxide was removed. Further, by desalinating and refining by dialysis membrane, the solution was freeze-dried, and 10.6 g of solubilized keratin powder derived from feathers (product 3 of the invention) was obtained.

Example 5: Manufacture of solubilized keratin (4)

In a beaker of inner volume of 1000 mL, 15 g of sodium hydroxide and 500 g of water were added, 30 g of ground feathers was supplied, and allowed to react for 5 minutes at room temperature. After the reaction, by dewatering the feathers, 60 g of alkali water and feathers (water content 50%) was prepared. In an Erlenmeyer flask of inner volume of 500 mL, 60 g of alkali water and features and 300 g of water were added, and hydrolyzed for 3 minutes at 120°C. After the hydrolysis, the same operation as in example 4 was performed, and 13. 8 g of solubilized keratin powder derived from feathers (product 4 of the invention) was obtained.

Example 6: Manufacture of solubilized keratin (5)

Washing 20 g of ground feathers in tap water, the feather weight after dewatering was adjusted to 40 g (water content 50%) . In an Erlenmeyer flask of inner volume of 500 mL, 2.4 g of sodium hydroxide and 300 g of water were added, 40 g of washed ground feathers was supplied, and hydrolyzed for 5 minutes at 120°C. After the hydrolysis, undecomposed matter was filtered, 4 g of 36% hydrochloric acid and 10.3 g of 30% hydrogen peroxide water were added, allowed to react for 18 hours in thermostatic oven at 45°C, 10 g of sodium sulfite was added, and excessive hydrogen peroxide was removed. Further, by desalinating and refining by a dialysis membrane, the solution was freeze-dried, and 8.6 g of solubilized keratin powder derived from feathers (product 5 of the invention) was obtained.

Example 7: Manufacture of solubilized keratin (6)

In 800 g of feathers adjusted to water content of 50%, 4000 g of 0.2N sodium hydroxide solutionwas added, and hydrolyzed for 15 minutes at 120°C. After the reaction, the solution was once cooled to room temperature, and 90 g of 36% hydrochloric acid was added thereto, the mixture was let stand overnight, and undecomposed matter was removed by filtering. Further, by desalinating and refining by ion exchange resin, the solution was spray-dried, and 88 g of solubilized keratin powder derived from feathers (product 6 of the invention) was obtained.

Example 8: Measurement of molecular weight

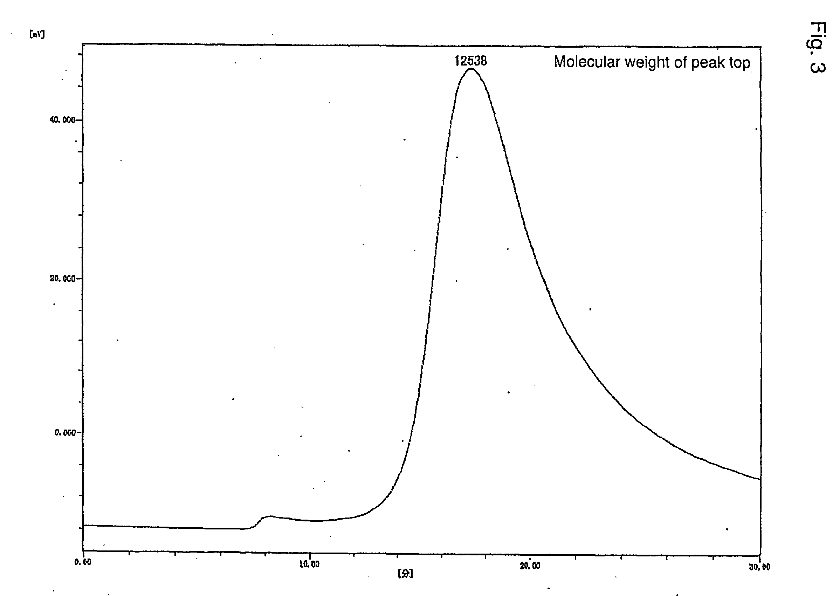

Keratin powders obtained in Examples 4 to 7 (products 3 to 6 of the invention) were dissolved to concentration of 25%, and the molecular weight of the solution was measured by gel filtration analysis, the results being shown in Table 1. Results of the gel filtration analysis of product 6 of the invention are shown in Fig. 3. Weight-average molecular weight by gel filtration analysis of the invention is measured in the following condition.

(Gel filtration analysis condition)

```
Column for gel filtration: G2000SWXL of Toso, 7.8 × 300
mm
```

Mobile phase: 0.1M phosphate buffer solution, pH 6.8 (containing 0.3M NaCl)

Flow velocity: 0.6 mL/min
Column temperature: 40°C
Detector: UV 230 nm
Molecular weight marker: Aldorase (Mw 158,000)
Bovine serum albumin (Mw 68000)
Ovalbumin (Mw 45000)
Kymotrypsinogen (Mw 25000)
Cytochrome C (Mw 12600)
Insulin (Mw 5500)

[Table 1]

|  | Product 3 | Product 4 | Product 5 | Product 6 |
|---|---|---|---|---|
| Weight-average molecular weight by gel filtration analysis | 12932 | 10383 | 10595 | 8464 |

As clear from results in Table 1, Fig. 3 and Example 2, the weight-average molecular weight by gel filtration analysis was confirmed to distribute in the range of 8000 to 13000.

Example 9: Water content effect test

Using 8.8 g of dry feathers, water content thereof was adjusted to 12%, 20%, 30%, 40%, 60%, 70%, and 80%, and 0.7 g of sodium hydroxide was added to each solution. Further, water was properly added until the entire water amount including feathers and water was 88 g, stirred well, and hydrolyzed for 5 minutes at 120°C. After the hydrolysis, the solution was centrifuged, the sediment was dried for 60 hours at 80°C, the weight of dried matter was measured, and the decomposition rate was compared by difference in water content of feathers. Results are shown in Table 2. As a result, raw materials of water content of 20% or more was found to be excellent in the rate of hydrolysis outstandingly as compared with raw materials of around 12%.

[Table 2]

| Water content (%) | 12 | 20 | 30 | 40 | 50 | 60 | 70 | 80 |
|---|---|---|---|---|---|---|---|---|
| Decomposition rate (%) | 47.5 | 76.1 | 79.5 | 77.5 | 75.6 | 78.0 | 80.0 | 73.3 |

Test example 1: Evaluation of coloration and smell

Solution stock for evaluation in the composition shown in Table 3 (effective ingredients 25%) was prepared, this solution was diluted 5 times in ion exchange water, the content of effective ingredients was adjusted to 5%, and color and smell of solubilized keratin solution of the invention were evaluated. By way of comparison, keratin hydrolyzate derived from wool (Promois WK of Seiwa Kasei) was diluted 5 times in ion exchange water, the content of effective ingredients was adjusted to 5%, and this solution was similarly evaluated.

[Table 3]

|  | Composition |
|---|---|
| Product 6 of the invention (powder) | 25 |
| 1,3-butylene glycol | 3 |
| Methyl paraoxy benzoate | 0.15 |
| Propyl paraoxy benzoate | 0.01 |
| Ion exchange water | Proper |
| Total | 100 |

Color and smell of each solution for evaluation were tested by a total of 20 panelists. Results are shown in Table 4. The numerals indicate the number of persons out of 20 panelists.

[Table 4]

|  | Product 6 is superior | Promois WK is superior | Undecided |
|---|---|---|---|
| Color | 2 | 2 | 16 |
| Smell | 12 | 3 | 5 |

As clear from the results in Table 4, mostpanelists smelled less in the solution of the product of the invention as compared with the comparative solution. That is, the solubilized keratin obtained by the method of the invention is substantially decreased in smell as compared with solubilized keratin prepared by conventional process.

Test example 2: Skin irritation test

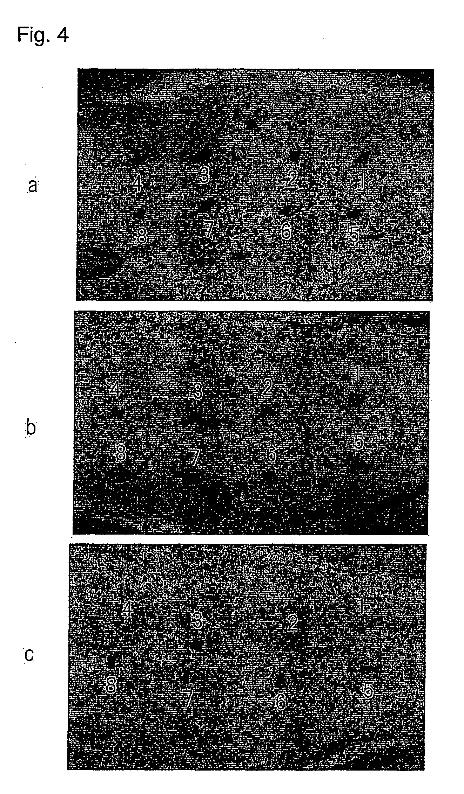

Skin irritation was tested in solubilized keratin prepared in example 1 (product 1 of the invention). Conforming to Guideline 2001 about safety evaluation of Japan Cosmetic Industry Association, skin sensitization was tested by using guinea pigs. A total of 15 guinea pigs were divided in three groups of 5 animals each, i.e., negative control group, positive control group, and test group, and the application position of sensitizing materials (respectively, purified water, dinitrochlorobenzene, product 1 of the invention) was clipped and marked with ink. Sensitizing materials except purified water were preliminarily diluted in purified water to specified concentration (wt%). Sensitizing materials were applied to the end of marked position on the skin of animals of each group, and the skin reaction was judged (see Fig. 4). Skin reaction was evaluated according to the criterion of skin reaction in the Guidance. Scoring is 0 when no red spot was found, and 1 when slight red spot was found, and the score got higher when the red color was increased, and the highest score was 4. For example, in the positive control group in Fig. 4b, an obvious red spot is noted, and the score in this case is 2. As a result, as shown in Fig. 4c, the test group of solubilized keratin of the invention was free from red spot at concentration of 0.01 to 2.0%, and the score for all animals was 0 (see Table 5), and the safety of the solubilized keratin of the invention in the specified concentration range was recognized. In some animals of test group of Fig. 4c of the solubilized keratin of the invention, red spots were partially noted, but the positions were different from the application positions of the solubilized keratin of the invention, and it is estimated to be caused by other reasons.

[Table 5]

| Animal No. | 2.0% | 1.0% | 0.5% | 0.2% | 0.1% | 0.05% | 0.02% | 0.01% |
|---|---|---|---|---|---|---|---|---|
| 1 | 0 | 0 | 0 | 0 | 0 | 0 | 0 | 0 |
| 2 | 0 | 0 | 0 | 0 | 0 | 0 | 0 | 0 |
| 3 | 0 | 0 | 0 | 0 | 0 | 0 | 0 | 0 |
| 4 | 0 | 0 | 0 | 0 | 0 | 0 | 0 | 0 |
| 5 | 0 | 0 | 0 | 0 | 0 | 0 | 0 | 0 |

Test example 3: Tensile strength test

About 1 g of 10 cm lock of hair (manufactured by Bulax) is bleached by immersing in 4.5% and 2.5% ammonia water mixed solution for 16 hours at room temperature. After bleaching, the hair is immersed for 10 minutes at 40°C in the same sample solutions as used in test example 1 (product 6 of the invention (5% solution), and Promois WK), washed in water, and dried by towel and drier. Distance between the hair and the drier is 15 cm. This process is repeated 6 times, and sample hair for evaluation is obtained. From this lock of hair, randomly, 15 hairs were selected, tested by creep meter (manufactured by Yamaden), tensile strength and hair diameter were measured, the tensile strength was divided by sectional area, and the average was determined. Results are shown in Table 6.

[Table 6]

| | Untreate d hair | Bleached hair | Bleached hair + water | Bleached hair + feather keratin | Bleached hair + wool keratin |
|---|---|---|---|---|---|
| Kgf/mm$^2$ | 35.95 | 31.32 | 30.18 | 39.18 | 36.56 |

**Claims**

1. A process for producing solubilized keratin, which comprises regulating the water content of keratin raw material to a hydrous state of 20 to 80%, hydrolyzing the same in an alkali solution, neutralizing the hydrolyzate liquid, and extracting a keratin hydrolyzate from the supernatant.

2. The process according to claim 1, wherein a cleaned keratin raw material is used.

3. The process according to claim 1 or 2, wherein the alkali concentration is 0.1 to 0.5 mol/L.

4. The process according to any one of claims 1 to 3, wherein the hydrolyzing process is executed in the condition of 80 to 120°C and 1 to 16 hours.

5. The process according to any one of claims 1 to 4, wherein peroxide is used in the neutralizing step.

**6.** The process according to any one of claims 1 to 5, wherein the keratin raw material is either wool or feathers.

**7.** The process according to claim 6, wherein the wool or feathers is clothes or bedding containing them, or waste wool or waste feathers.

**8.** A solubilized feather keratin having an average molecular weight of 8000 to 13000 (gel filtration method) and manufactured by the process according to any one of claims 1 to 7 using feathers as keratin raw material.

**9.** A cosmetic blend composed of the solubilized keratin manufactured by the process according to any one of claims 1 to 8.

Fig. 1

Tank CIP inlet

Raw material

Water

Caustic soda

M

TI

P

Fig. 2

UF membrane

Fig. 3

Fig. 4

a

b

c

## INTERNATIONAL SEARCH REPORT

| | International application No. |
|---|---|
| | PCT/JP2005/005672 |

A. CLASSIFICATION OF SUBJECT MATTER
   Int.Cl⁷  C07K1/14//A61K7/06, 7/075, 7/08, 7/50

According to International Patent Classification (IPC) or to both national classification and IPC

B. FIELDS SEARCHED

Minimum documentation searched (classification system followed by classification symbols)
   Int.Cl⁷  C07K1/14//A61K7/06, 7/075, 7/08, 7/50

Documentation searched other than minimum documentation to the extent that such documents are included in the fields searched

Electronic data base consulted during the international search (name of data base and, where practicable, search terms used)
   CA/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X | JP 57-163392 A  (Kao Soap Co., Ltd.),<br>07 October, 1982 (07.10.82),<br>Full text<br>(Family: none) | 1-9 |

☐ Further documents are listed in the continuation of Box C.   ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>12 April, 2005 (12.04.05) | Date of mailing of the international search report<br>10 May, 2005 (10.05.05) |
|---|---|
| Name and mailing address of the ISA/<br>   Japanese Patent Office | Authorized officer |
| Facsimile No. | Telephone No. |

Form PCT/ISA/210 (second sheet) (January 2004)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4281856 A
- JP 4312534 A
- JP 2001302800 A
- JP 61002416 A
- JP 57163392 A
- JP 5170926 A
- JP 6336499 A
- JP 6100600 A
- JP 6116300 A
- JP 10291998 A
- JP 10291999 A
- JP 7021061 A
- JP 2777196 B
- JP 3283302 B
- JP 2003301377 A